# EUROPÄISCHE PATENTSCHRIFT

(11) **EP 1 558 150 B1**
(45) Veröffentlichungstag und Bekanntmachung des Hinweises auf die Patenterteilung: **22.03.2006**
(21) Anmeldenummer: 02785363.9
(22) Anmeldetag: 05.11.2002
(51) Int. Cl.: A61B 17/15, A61B 5/11, A61F 2/46, A61B 19/00

(54) **VORRICHTUNG ZUR BESTIMMUNG DER LAGE EINER KNIEGELENKENDOPROTHESE**
DEVICE FOR DETERMINING THE POSITION OF A KNEE-JOINT ENDOPROSTHESIS
DISPOSITIF POUR DETERMINER LA POSITION D'UNE ENDOPROTHESE DU GENOU

(43) Veröffentlichungstag der Anmeldung: 03.08.2005
(73) Patentinhaber: Aesculap AG & Co. KG, 78532 Tuttlingen (DE)
(72) Erfinder: FRIEDRICH, Dirk, 78532 Tuttlingen (DE); LEITNER, François, F-38410 Uriage (FR)
(74) Vertreter: Boehme, Ulrich
(86) Internationale Anmeldenummer: PCT/EP2002/012319
(87) Internationale Veröffentlichungsnummer: WO 2004/041097

(56) Entgegenhaltungen:
- DE-A- 10 031 887
- US-A- 5 682 886
- US-A1- 2002 038 085
- US-A1- 2002 107 522

## Beschreibung

Die Erfindung betrifft ein System zur Bestimmung der Lage des Tibiateils und/oder des Femurteils einer Kniegelenkendoprothese relativ zum proximalen Tibiakopf bzw. zum distalen Femur mit einem Navigationssystem zur Überwachung der Lage des Femurs und der Tibia über an diesen festlegbare Markierelemente, mit einem Distraktionsgerät, welches den distalen Femur und den proximalen Tibiakopf bei gestrecktem und bei abgewinkeltem Knie lateral und medial mit einer definierten Kraft in eine gespreizte Position verschiebt, mit einer Datenverarbeitungsanlage, die die Relativpositionen von Femur und Tibia bei dieser Distraktion und damit die Größe des Spaltes zwischen Femur und Tibia bestimmt und die in Abhängigkeit von geometrischen Daten der Kniegelenkendoprothese und verschiedenen angenommenen Positionen des Tibiateils an der Tibia und/oder des Femurteils am Femur verschiedene virtuelle Relativpositionen von Femur und Tibia bei gestrecktem und gebeugtem Knie berechnet.

Bei der Implantatation einer Kniegelenkendoprothese werden häufig Endoprothesen verwendet, die aus zwei oder drei Teilen bestehen, beispielsweise aus einem an der Tibia festlegbaren Tibiateil, einem am Femur festlegbaren Femurteil und einem zwischen Femurteil und Tibiateil angeordneten Zwischenteil. Tibiateil und Femurteil müssen mit der Tibia bzw. dem Femur so verbunden werden, daß die Kinematik des ursprünglichen Kniegelenks möglichst gut reproduziert wird. Um dies zu erreichen, müssen der Tibiakopf und der Femur durch Sägeschnitte entsprechend reseziert werden, so daß Tibiateil und Femurteil in der gewünschten Position an Tibia und Femur zur Anlage kommen, diese Anlageposition ist verantwortlich für die optimale Kinematik des Kniegelenks.

Es ist bekannt, zur Implantatation eines Kniegelenkes die ursprüngliche Kinematik des Knies und auch die Kinematik des Knies nach der Implantatation der Endoprothese dadurch zu überwachen, daß sowohl der Femur als auch die Tibia durch ein an sich bekanntes Navigationssystem lageüberwacht werden. Ein solches Navigationssystem kann die Position bestimmter Markierelemente im Raum laufend bestimmen, bei den Markierelementen kann es sich beispielsweise um Elemente mit mehreren strahlenden Sendern handeln, etwa Infrarotdioden, in anderen Fällen handelt es sich dabei um Markierelemente mit mehreren im Abstand zueinander angeordneten Reflexionskörpern, die z.B. eine Infrarotstrahlung gut reflektieren. Das Navigationssystem erfaßt die Strahlung, die von dem Markierelement ausgeht, und kann dadurch die Lage des Markierelementes im Raum bestimmen und damit auch die Lage des Körperteils, an dem das Markierelement festgelegt ist. Derartige Navigationssysteme werden bei Knieoperationen regelmäßig eingesetzt, um den Bewegungsablauf von Femur und Tibia zu überwachen und auch um anatomischgeometrische Daten des Femurs und des Tibiakopfes mittels geeigneter navigierter Anlage- oder Tastinstrumente zu erfassen (DE 100 31 887 A1).

Für das Gelingen einer Knieoperation ist wichtig, daß nach dem Einsetzen des Implantates Femur und Tibia auf der lateralen und medialen Seite gleichmäßig durch die Femur und Tibia verbindenden Bänder gegeneinander gespannt werden, und zwar möglichst sowohl in der gestreckten als auch in der abgewinkelten Stellung des Beines. Um dies zu erreichen ist es bekannt, Probeimplantate einzusetzen und nach dem Einsetzen der Probeimplantate die sich für diese Probeimplantate ergebenden Spannungen zu messen, beispielsweise mit Hilfe eines Distraktionsgerätes, welches die zusammenhaltenden Kräfte durch Aufspreizen des Spaltes zwischen Femur und Tibia mißt.

Dieses Verfahren ist außerordentlich umständlich und birgt die Gefahr in sich, daß aufgrund von Fehlmessungen nach erfolgter Operation doch nicht die erwünschten Spannungsverhältnisse erreicht werden, dies kann dazu führen, daß die Gelenkflächen nach der Operation zu wenig oder aber umgekehrt auch zu stark gegeneinander gespannt werden.

Es ist Aufgabe der Erfindung, ein System der gattungsgemäßen Art so auszubilden, daß während des Operationsablaufes und ohne Veränderungen an dem ursprünglichen Kniegelenk vorzunehmen, bestimmt werden kann, wie die Teile der Endoprothese zu implantieren sind, um die natürlichen Spannungsverhältnisse im Kniegelenk in der gewünschten Weise zu reproduzieren oder gegebenenfalls auch abzuwandeln.

Diese Aufgabe wird erfindungsgemäß gelöst durch ein System zur Bestimmung der Lage des Tibiateiles und/oder des Femurteils einer Kniegelenkendoprothese relativ zum proximalen Tibiakopf bzw. zum distalen Femur mit einem Navigationssystem zur Überwachung der Lage des Femurs und der Tibia über an diesen festlegbare Markierelemente, mit einem Distraktionsgerät, welches den distalen Femur und den proximalen Tibiakopf bei gestrecktem und bei abgewinkeltem Knie lateral und medial mit einer definierten Kraft in eine gespreizte Position verschiebt, mit einer Datenverarbeitungsanlage, die die Relativpositionen von Femur und Tibia bei dieser Distraktion und damit die Größe des Spaltes zwischen Femur und Tibia bestimmt und die in Abhängigkeit von geometrischen Daten der Kniegelenkendoprothese und verschiedenen angenommenen Positionen des Tibiateils an der Tibia und/oder des Femurteils am Femur verschiedene virtuelle Relativpositionen von Femur und Tibia bei gestrecktem und gebeugtem Knie berechnet.

Mit diesem System kann damit die Lage des Tibiateils und/oder des Femurteils bestimmt werden, ohne daß an Tibia oder Femur Veränderungen vorgenommen werden müssen. Es genügt, das Kniegelenk freizulegen und dann durch Aufspreizen des Spaltes zwischen Tibiakopf und Femur bei gestrecktem Knie und bei abgebeugtem Knie die jeweils sich einstellende Relativposition von Femur und Tibia und damit die Spaltbreite zwischen Tibiakopf und Femur zu bestimmen. In einem nächsten Schritt werden für die ausgewählten Prothesenteile bestimmte Positionen an der Tibia bzw. am Femur festgelegt. Dies erfolgt nur virtuell, also nicht tatsächlich, indem Anlageflächen bestimmt werden, an denen die Prothesenteile an Femur und Tibia angelegt werden können, beispielsweise Sägeebenen.

Mit den so angenommenen Anlageflächen und damit den so angenommenen Positionen der Implantatteile an Femur und/oder Tibia kann dann berechnet werden, wie sich Femur und Tibia bei der Beugebewegung relativ zueinander bewegen, sofern bei dieser Berechnung die geometrischen Daten der Implantatteile zugrunde gelegt werden. Diese geometrischen Daten geben an, wie die Implantatteile sich relativ zueinander bewegen, und aus den angenommenen Positionen der Implantatteile an Femur und Tibia einerseits und der Relativbewegung der Implantate zueinander andererseits lassen sich die virtuellen Relativpositionen von Femur und Tibia während der Beugebewegung berechnen.

Diese Positionsdaten sind deswegen virtuell, weil tatsächlich kein Prothesenteil bewegt wird, sondern diese Bewegung erfolgt durch eine reine Berechnung.

Die virtuellen Positionsdaten für eine bestimmte angenommene Position der Prothesenteile werden anschließend verglichen mit den Relativpositionen, die bei dem anatomischen Kniegelenk bei der Aufspreizung mit Hilfe des Distraktionsgerätes aufgenommen worden sind.

Eine optimale Reproduktion der körpereigenen Kinematik läßt sich dann erreichen, wenn gemäß einer bevorzugten Ausführungsform eine ausgewählte Position so bestimmt wird, daß die virtuelle Relativposition von Femur und Tibia mit der gespreizten Position übereinstimmt. Es ergibt sich dann ein Bewegungsablauf, der dem natürlichen Bewegungsablauf entspricht, wobei in gestreckter und in gebeugter Stellung die Spannung des Bandapparates identisch mit dem natürlichen Zustand ist.

Es ist aber natürlich auch möglich, daß der Operateur gezielt eine Abweichung der virtuellen Relativposition von der tatsächlichen Relativposition wünscht, beispielsweise zur Korrektur einer Fehlstellung. Dann besteht die Möglichkeit, durch Variation der angenommenen Position der Implantatteile solange virtuelle Relativpositionen zu errechnen, bis eine virtuelle Relativposition gefunden ist, die der gewünschten Abweichung von der tatsächlichen Relativposition entspricht, mit anderen Worten wird die Bewegung des Knies auf diese Weise in Abhängigkeit von unterschiedlich angenommen Positionen der Prothesenteile simuliert, ohne daß dazu eine Bearbeitung des Tibiakopfes oder des Femurs notwendig wäre.

Beispielsweise kann vorgesehen sein, daß man eine ausgewählte Position so bestimmt, daß die Größe des Spaltes zwischen Femur und Tibia in der abgewinkelten Stellung und/oder der gestreckten Stellung des Knies lateral und medial zumindest annähernd gleich ist.

Die Position der Prothesenteile kann in vielfältiger Weise unterschiedlich angenommen werden, beispielsweise kann bei einer ersten bevorzugten Ausführungsform vorgesehen sein, daß man zur Berechnung verschiedener virtueller Relativpositionen die angenommene Position des Femurteils durch dessen in sich parallele Verschiebung senkrecht zur Längsachse des Femurs verschiebt. Die Längsachse des Femurs kann dabei identisch sein mit der mechanischen Achse des Femurs, die das Kniegelenk mit dem Hüftgelenk verbindet. Diese unterschiedliche Relativposition entspricht einer unterschiedlichen Dicke der am distalen Ende des Femurs resezierten Gelenkfläche.

Bei einer weiteren bevorzugten Ausführungsform ist vorgesehen, daß man zur Berechnung verschiedener virtueller Relativpositionen die angenommene Position des Femurteils durch dessen in sich parallele Verschiebung in anterior-posteriorer Richtung verschiebt. Mit einer solchen Verschiebung ergibt sich eine Abstandsänderung der dorsalen Gelenkfläche des Femurteils, so daß diese Gelenkfläche bei gebeugtem Knie verschiedene Abstände zum Tibiakopf aufweist.

Bei einer weiteren bevorzugten Variation der angenommenen Position des Femurteils wird vorgesehen, daß man zur Berechnung verschiedener virtueller Relativpositionen die angenommene Position des Femurteils durch dessen Verschwenkung um eine anterior-posterior verlaufende Achse verändert. Eine solche Verschwenkung führt zu einer Korrektur der Varus-Valgus-Stellung des Knies, d.h. dadurch wird auch bei gestrecktem Knie die Spaltbreite zwischen Femur und Tibiateil lateral und medial verändert.

Eine weitere Möglichkeit einer Veränderung der angenommen Position sieht vor, daß man zur Berechnung verschiedener virtueller Relativpositionen die angenommene Position des Femurteils durch dessen Verschwenkung um eine medial-lateral verlaufende Achse verändert. Dies führt zu einer Neigung der Gelenkflächen gegenüber der Längsachse des Femurs und kann für bestimmte Korrekturen von Bedeutung sein.

Die genannten Variationen können einzeln oder in Kombination vorgenommen werden, und es ist klar, daß jede dieser Änderungen der Position des Femurteils zu einem geänderten kinematischen Verhalten des Kniegelenks führt. Durch die beschriebene Simulation dieser Bewegung, die sich durch die Berechnung der jeweils entsprechenden virtuellen Relativpositionen von Femur und Tibia ergeben, kann der Operateur die Konsequenzen einer Änderung der Position in jedem Falle am Vergleich der virtuellen Relativpositionen mit der gespreizten Position im gestreckten und abgewinkelten Knie feststellen und die angenommene Position so lange variieren, bis einerseits die gewünschte Übereinstimmung mit der gespreizten Position möglichst gut erreicht wird und andererseits eine gegebenenfalls erwünschte Korrektur, beispielsweise eine Varus-Valgus-Korrektur.

Zusätzlich zu unterschiedlich angenommenen Positionen ist es auch gemäß einer Weiterbildung der Erfindung möglich, daß man zur Berechnung verschiedener virtueller Relativpositionen von unterschiedlich dimensionierten Tibiateilen und/oder Femurteilen ausgeht. Es ist also möglich, verschieden große Tibia- oder Femurteile bei dieser Simulation anzunehmen und bei der Simulation deren Auswirkungen auf die virtuellen Relativpositionen zu berechnen. Der Operateur hat damit mit der Wahl der Abmessungen der Tibia- oder Femurteile einerseits und der Variation der Position dieser Teile andererseits eine Vielzahl von Möglichkeiten zur Verfügung, die Kniekinematik zu beeinflussen, und das Ergebnis dieser unterschiedlichen Parameter kann durch die beschriebene Simulation während der Operation vorab überprüft werden, ohne daß bereits Tibia oder Femur bearbeitet werden müssen.

Es ist vorteilhaft, wenn man bei der Bestimmung der gespreizten Position den Spalt zwischen Femur und Tibiakopf maximal aufweitet. Der Bandapparat wird beim Aufweiten zunehmend steifer und ist ab einer bestimmten Dehnung praktisch nicht mehr weiter dehnbar, d.h. die Distraktion läuft in eine Sättigung ein. Die erzielbare Dehnung ist in diesem Bereich relativ unabhängig von der aufgewandten Dehnungskraft, und aus diesem Grunde ist es vorteilhaft, bis in diesen Bereich zu distrahieren, man erhält dadurch gut reproduzierbare Ergebnisse.

Aus der Überwachung der Position von Femur und Tibia läßt sich bei der Beugungsbewegung des Knies und auch bei Anlage des Distraktionsgerätes der tatsächliche Spalt zwischen den Gleitflächen des Femurs und dem Tibiakopf nur dann genau bestimmen, wenn zusätzlich geometrische Daten über die Form von Femur und Tibia vorliegen. Es ist daher gemäß einer weiteren Fortbildung der Erfindung vorgesehen, daß man zur Bestimmung der Größe des Spaltes zwischen Femur und Tibia die Kontur des proximalen Tibiakopfes und die Kontur des distalen Femurs durch die Anlage von mindestens einem navigierten Anlageelement an diese Konturen bestimmt. Dies ist eine an sich bekannte Technik zur Konturbestimmung, durch die Anlage eines navigierten Anlageelementes kann das Navigationssystem durch die jeweilige Lage des Anlageelementes relativ zu Femur und Tibia den Anlagepunkt oder die Anlagelinie genau lokalisieren, und daraus lassen sich geometrische Daten für die Gesamtkontur des Tibiakopfes und des Femurs errechnen.

Beispielsweise kann vorgesehen sein, daß ein Anlageelement eine Tastspitze aufweist, mit der verschiedene Punkte der Konturen abgetastet werden.

Bei einer anderen Ausgestaltung ist vorgesehen, daß ein Anlageelement eine ebene Anlagefläche aufweist, die man an die zu bestimmende Kontur anlegt.

Bei einer besonders bevorzugten Ausführungsform ist vorgesehen, daß ein Anlageelement zwei senkrecht aufeinander stehende Anlageflächen aufweist, die man gemeinsam an die zu bestimmende Kontur anlegt. Dies ist beispielsweise günstig zur Bestimmung der Kontur der Kondylenflächen des Femurs, die beiden Anlageflächen können dann an die distale Kondylenfläche bzw. die dorsale Kondylenfläche angelegt werden, so daß Informationen über die Konturen in diesem Bereich gewonnen werden können.

Der Vergleich der gespreizten Position und der jeweils durch die Simulation errechneten virtuellen Relativposition wird vorzugsweise mit Hilfe eines Sichtgerätes oder einer Anzeige erfolgen, auf der dem Operateur aus diesen Daten abgeleitete Informationen angezeigt werden, beispielsweise kann gemäß einer bevorzugten Ausführungsform vorgesehen sein, daß man auf einer Anzeige die Größe des medialen und lateralen Spaltes in der gestreckten und in der abgewinkelten Stellung des Knies in den jeweils berechneten virtuellen Relativpositionen darstellt. Der Operateur kann jetzt beispielsweise die angenommen Positionen so verändern, daß die Größe des Spaltes medial und lateral und nach Möglichkeit in gestrecktem und abgewinkeltem Zustand gleich wird.

Weiterhin ist es günstig, wenn man auf einer Anzeige die für die Bestimmung verschiedener virtueller Relativpositionen angenommene Position des Femurteiles relativ zu geometrischen Daten des Femurs anzeigt, beispielsweise zu einem den geometrischen Daten entsprechenden Bild des Femurs. Der Operateur kann dann beispielsweise die Neigung einer Anlagefläche des Femurteils relativ zur Längsachse des Femur oder ein ähnliches Maß unmittelbar ablesen und eine entsprechende Variation der angenommenen Position so vornehmen, daß diese abgelesene Größe den Vorstellungen des Operateurs entspricht.

Während es ohne weiteres möglich ist, das beschriebene Verfahren bei unbearbeitetem Tibiakopf und unbearbeitetem Femur durchzuführen, kann es in bestimmten Fällen vorteilhaft sein, wenn man vor der Aufspreizung des Spaltes zwischen Femur und Tibiakopf die proximale Gelenkfläche des Tibiakopfes längs einer Ebene reseziert, die senkrecht auf der Längsachse der Tibia steht. Man erhält dadurch eine Anlagefläche für das Distraktionsgerät und kann diese Anlagefläche auch als Anlagefläche für das Tibiateil verwenden, so daß die Anpassung der Endoprothese dann ausschließlich im Femurteil erfolgt.

Es kann vorgesehen sein, daß der Datenverarbeitungsanlage eine Anzeige zugeordnet ist, die der Relativposition von Femur und Tibia bei der Distraktion entsprechende Daten und den virtuellen Relativpositionen entsprechende Daten zum Zwecke von deren Vergleich anzeigt. Insbesondere kann dabei vorgesehen sein, daß die Datenverarbeitungsanlage auf der Anzeige die Größe des medialen und lateralen Spaltes in der gestreckten und in der abgewinkelten Stellung des Knies in den jeweils berechneten virtuellen Relativpositionen darstellt.

Es ist weiterhin günstig, wenn die Datenverarbeitungsanlage auf der Anzeige die für die Bestimmung verschiedener virtueller Relativpositionen angenommene Position des Femurteiles relativ zu geometrischen Daten des Femurs anzeigt.

Gemäß einer bevorzugten Ausführungsform umfaßt eine derartige Vorrichtung mindestens ein navigiertes Anlageelement, welches zur Bestimmung der Größe des Spaltes zwischen Femur und Tibia an die Kontur des proximalen Tibiakopfes und die Kontur des distalen Femurs anlegbar ist.

Es kann dabei vorgesehen sein, daß ein Anlageelement eine Tastspitze aufweist, mit der verschiedene Punkte der Konturen abtastbar sind.

Bei einer anderen Ausführungsform ist vorgesehen, daß ein Anlageelement eine ebene Anlagefläche aufweist, die man an die zu bestimmende Kontur anlegt.

Das Anlegeelement kann dabei zwei senkrecht aufeinanderstehende Anlageflächen aufweisen, die man gemeinsam an die zu bestimmende Kontur anlegt.

Die nachfolgende Beschreibung bevorzugter Ausführungsformen der Erfindung dient im Zusammenhang mit der Zeichnung der näheren Erläuterung. Es zeigen:

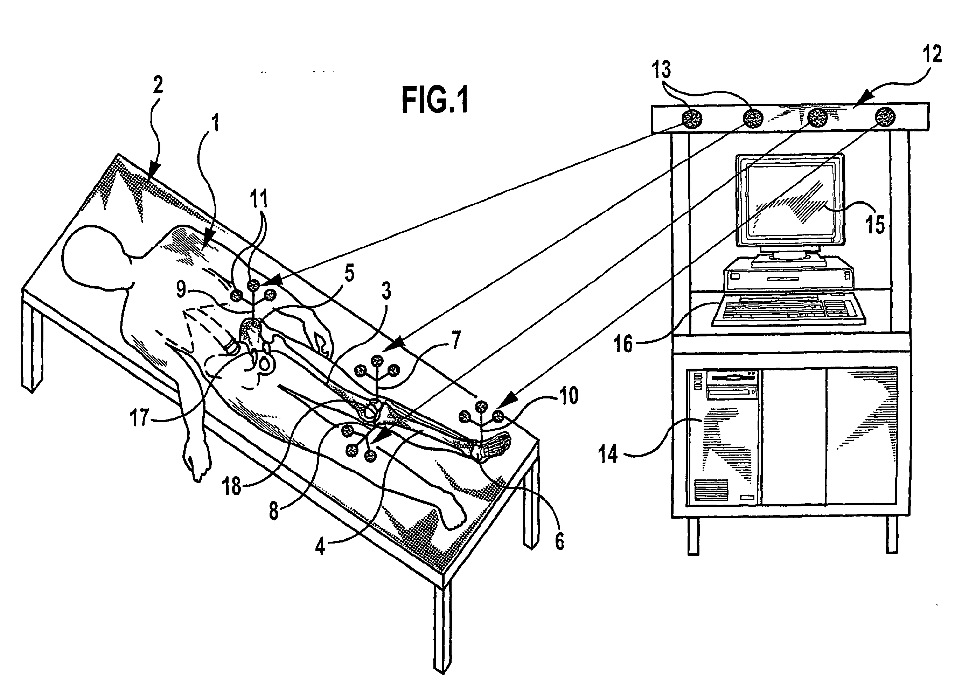

- Figur 1:: eine schematische Gesamtansicht eines Systems zur Bestimmung der Lage von Prothesenteilen im Knie eines Patienten;
- Figur 2:: eine schematische Vorderansicht eines abgewinkelten Knies mit einem navigierten Femur, einer navigierten Tibia und einer navigierten Anlageplatte an einer resezierten Tibiafläche;
- Figur 3:: eine seitliche Ansicht eines navigierten Femurs mit einem navigierten Anlageelement mit zwei senkrecht zueinander stehenden Anlageflächen und mit einem navigierten Anlageelement in Form einer Tastspitze;
- Figur 4:: eine Ansicht ähnlich Figur 2 mit einem zwischen Femur und Tibiakopf eingesetzten Distraktionsgerät;
- Figur 5:: eine Seitenansicht des abgewinkelten Kies der Figur 4;
- Figur 6:: eine Ansicht ähnlich Figur 4 bei einem Kniegelenk in der distrahierten Position ohne Darstellung des Distraktionsgerätes;
- Figur 7:: eine Ansicht ähnlich Figur 6 bei einem gestreckten Knie;
- Figur 8:: eine Anzeige für die mediale und laterale Spaltbreite bei abgewinkeltem und bei gestrecktem Knie;
- Figur 9:: eine Vorderansicht eines Femurs mit in Richtung der Längsachse des Femurs verschoben angenommen Positionen des Femurteils einer Endoprothese;
- Figur 10:: eine Ansicht ähnlich Figur 9 mit einer um eine anterior-posteriorer Achse verschwenkten Anlagefläche;
- Figur 11:: eine Seitenansicht eines Femurs mit einer um eine medial-laterale Achse verschwenkten Anlagefläche;
- Figur 12:: eine Ansicht ähnlich Figur 11 mit einer in anterior-posteriore Richtung verschobenen Anlagefläche;
- Figur 13:: eine schematische Seitenansicht eines Kniegelenks mit eingesetzter Endoprothese und
- Figur 14:: ein Flußdiagramm zur Beschreibung des Verfahrensablaufes bei der Bestimmung der Lage der Endoprothesenteile relativ zu Tibia und Femur.

Zum Ersetzen eines Kniegelenkes durch eine Kniegelenkendoprothese wird der Patient 1 auf einem Operationstisch 2 gelagert, und das Kniegelenk wird in an sich bekannter Weise eröffnet. Zumindest am Femur 3 und an der Tibia 4 und vorzugsweise auch am Hüftknochen 5 und am Fuß 6 des Patienten werden Markierelemente 7, 8, 9 bzw. 10 starr festgelegt, beispielsweise durch Einschrauben einer Knochenschraube. Jedes dieser Markierelemente trägt drei im Abstand zueinander angeordnete Emitter 11, dies können aktive Strahlungssender für Ultraschallstrahlung, Infrarotstrahlung oder eine ähnliche Strahlung sein oder auch passive Reflexionselemente für eine solche Strahlung, die dann auf sie auffallende Strahlung reflektieren und damit emittieren. Diese Markierelemente arbeiten zusammen mit einem Navigationssystem 12 mit mehreren Strahlungsempfängern 13, welches die Lage und Orientierung der Markierelemente im Raum feststellt und dieser Lage entsprechende Daten einer Datenverarbeitungsanlage 14 zuführt. Die Datenverarbeitungsanlage 14 ist mit einer Anzeige 15 in Form eines Bildschirmes ausgestattet und mit einer Tastatur 16 zur Eingabe von zusätzlichen Daten.

Zur Vorbereitung einer Implantatation einer Kniegelenkendoprothese werden zunächst die mechanischen Achsen des Femurs 3 und der Tibia 4 bestimmt. Diese mechanische Achse des Femurs 3 ergibt sich beispielsweise aus der Verbindungslinie des Hüftgelenks 17 und des Kniegelenks 18, diese Gelenke lassen sich mit Hilfe des Navigationssystemes 12 in an sich bekannter Weise dadurch bestimmen, daß Femur und Hüftgelenk einerseits sowie Femur und Tibia andererseits gegeneinander bewegt werden, in gleicher Weise kann die mechanische Achse der Tibia als Verbindungslinie des Fußgelenkes und des Kniegelenkes durch Relativbewegung von Femur und Tibia einerseits und Tibia und Fuß andererseits bestimmt werden. Durch diese Bewegung werden auch die mit Femur, Tibia, Hüftknochen und Fuß starr verbundenen Markierelemente auf durch die Gelenke definierten Bahnen bewegt, und aus diesen Bahnen kann die Datenverarbeitungsanlage 14 dann die Lage der Gelenke relativ zu den Markierelementen und damit auch zu den Körperteilen bestimmen, mit denen die Markierelemente fest verbunden sind.

Man erhält auf diese Weise je eine mechanische Achse des Femurs und der Tibia, die annähernd der Längsrichtung dieser Knochen entsprechen.

In einem ersten Operationsschritt wird nach Bestimmung der mechanischen Achsen von Femur und Tibia der Tibiakopf 19 vorbereitet, also das proximale Ende der Tibia. Die dem Femur 20, also dem distalen Ende des Femurs 3, zugewandte Gelenkfläche wird durch einen ebenen Sägeschnitt entfernt, die sich dadurch ergebende Sägeebene 21 verläuft senkrecht zu der zuvor bestimmten mechanischen Achse der Tibia und befindet sich in der Regel nur einige Millimeter unterhalb des proximalen Endes des Tibiakopfes 19.

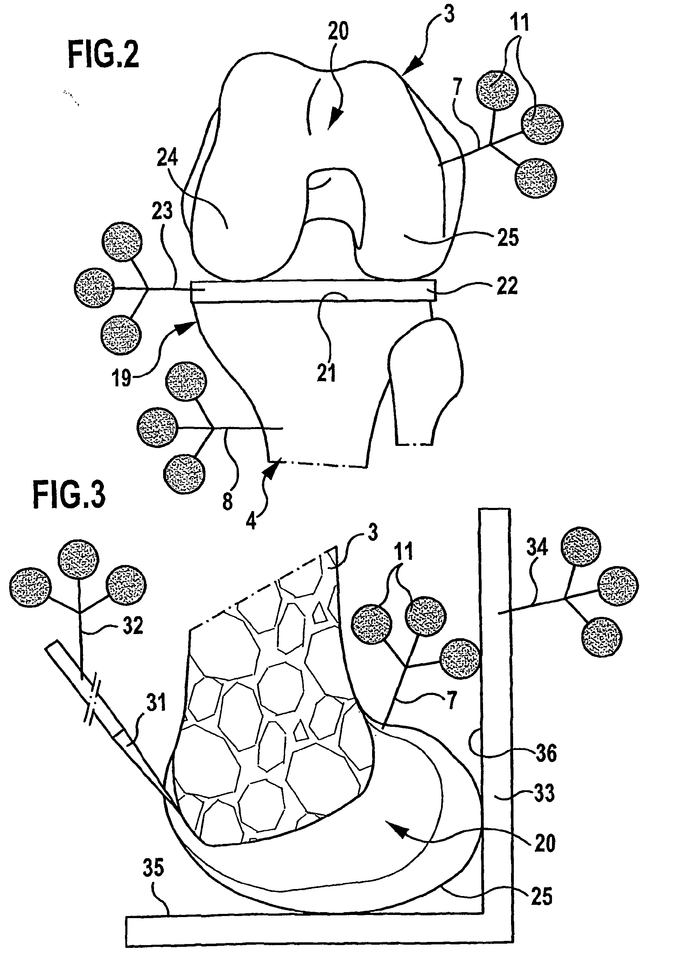

Die genaue Lage der Sägeebene 21 wird noch einmal dadurch kontrolliert, daß auf die Sägeebene 21 ein plattenförmiges Anlageelement 22 aufgelegt wird, welches seinerseits starr mit einem Markierelement 23 verbunden wird, so daß das Navigationssystem die Lage des Anlageelementes 22 im Raum und damit auch die Lage der Sägeebene 21 bestimmen kann (Figur 2).

In einem nächsten Schritt wird in den Zwischenraum zwischen der Sägeebene 21 des Tibiakopfes 19 einerseits und der nebeneinander und im Abstand voneinander verlaufenden Gelenkflächen 24, 25 des Femurs 20 andererseits ein Distraktionsgerät 26 eingeschoben. Dieses weist zwei relativ zueinander verschiebbare Spreizglieder 27, 28 auf, ein unteres stegförmiges Spreizglied 27 wird an die Sägeebene 21 angelegt, ein oberes parallel dazu verlaufendes Spreizglied 28 an eine der beiden Gelenkflächen 24, 25. An jeder dieser Gelenkflächen 24, 25 wird ein eigenes Distraktionsgerät 26 angelegt, so daß beim Aufspreizen der beiden Spreizglieder 27, 28 eines Distraktionsgeräts 26 jeweils die eine oder die andere Gelenkfläche 24 bzw. 25 von der Sägeebene 21 entfernt werden kann.

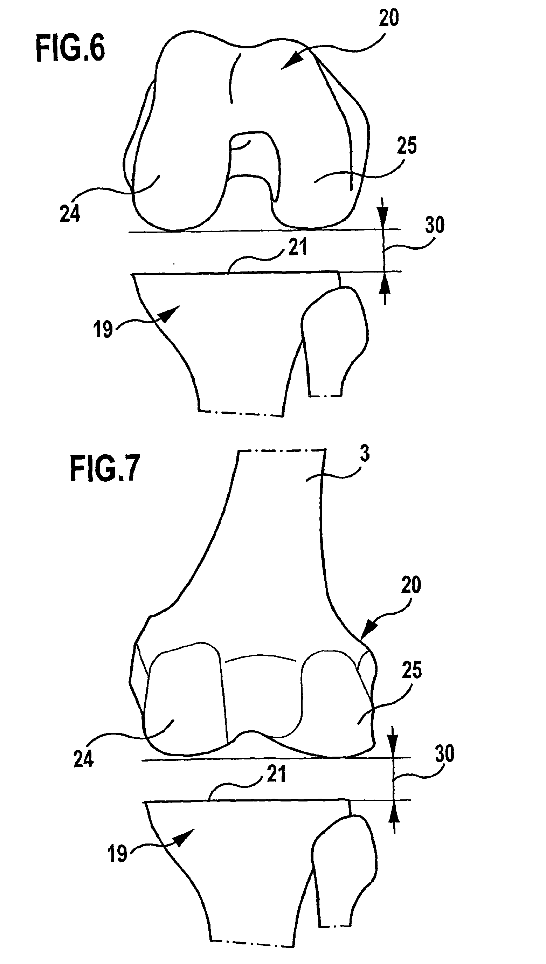

Die Konstruktion des Distraktionsgerätes kann sehr unterschiedlich sein, es gibt Distraktionsgeräte dieser Art in großer Zahl, wichtig ist nur, daß es damit gelingt, durch Verschieben der Spreizglieder 27, 28 gegeneinander, beispielsweise mittels eines an diesen angreifenden, in Figur 5 nur sehr schematisch dargestellten Spreizinstrumentes 29, den Zwischenraum zwischen Gelenkflächen 24, 25 einerseits und Sägeebene 21 andererseits zu vergrößern.

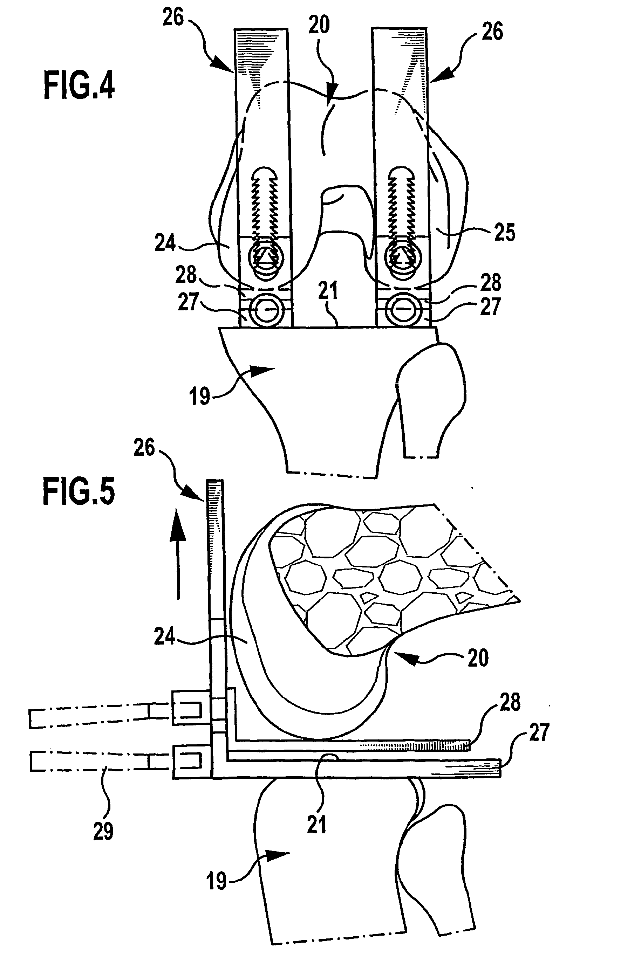

Dieser Vergrößerung wird von den Femur und die Tibia seitlich verbindenden Seitenbändern ein Widerstand entgegengesetzt, diese Seitenbänder spannen das distale Femurende und das proximale Tibiaende gegeneinander. Gegen die Kraft dieser Bänder erfolgt das Aufspreizen des Zwischenraumes zwischen Femur und Tibiakopf, und zwar sowohl bei abgewinkeltem Knie, wie es in den Figuren 4 und 5 dargestellt ist, als auch bei gestrecktem Knie. Die Seitenbänder weisen beim Beginn der Dehnung ein annähernd elastisches Verhalten auf, die Dehnfähigkeit erreicht jedoch einen Sättigungswert, so daß beim Aufbringen einer einen bestimmten Wert überschreitenden Kraft in jedem Falle eine maximale Dehnung der Seitenbänder zu erreichen ist und damit ein maximaler Spalt zwischen Femur einerseits und Tibiakopf andererseits.

In Figur 6 ist dieser maximale Spalt 30 schematisch bei einem abgewinkelten Knie dargestellt, in Figur 7 bei einem gestreckten. Die Spaltbreite wird definiert durch den Abstand der beiden Gelenkflächen 24, 25 des Femurs 3 von der Sägeebene 21. In der Darstellung der Figur 6 und 7 ist die Spaltbreite bei beiden Gelenkflächen 24, 25 etwa gleich, dies muß aber keineswegs so sein, es kann aufgrund von Fehlstellungen oder von Verwachsungen durchaus unterschiedliche Spaltbreiten bei den beiden Gelenkflächen 24, 25 geben, und zwar sowohl bei abgewinkeltem Knie als auch bei gestrecktem Knie.

Die Relativposition des Femurs und der Tibia, die diese sowohl bei abgewinkeltem als auch bei gestrecktem Knie nach der Spreizung der Distraktionsgeräte 26 einnehmen, werden vom Navigationssystem 12 bestimmt und in der Datenverarbeitungsanlage 14 werden entsprechende Datensätze gespeichert, diese repräsentieren also die Kinematik des zu ersetzenden Kniegelenks, die so erzielten Spaltbreiten geben Auskunft über die Spannung der beiden Seitenbänder.

Wenn sich bei dieser Untersuchung sehr unterschiedliche Werte der Spannung der Seitenbänder ergeben, hat der Operateur bereits jetzt die Möglichkeit, durch kleine Einschnitte an einem der Seitenbänder dessen Spannungsverhalten zu ändern und dadurch eine gewünschte Korrektur vorzunehmen, nach jeder Korrektur wird erneut eine Distraktion und eine Bestimmung der Relativpositionen von Femur und Tibiakopf vorgenommen, so daß das Ergebnis dieser Korrektur sofort festgestellt werden kann.

Für das weitere Verfahren werden auch geometrische Daten über die Form des Femurs benötigt. Diese Daten lassen sich mit Hilfe von navigierten Anlageelementen erreichen, also Anlageelementen, die jeweils mit einem Markierelement versehen sind. Ein solches Anlageelement kann beispielsweise eine Tastspitze 31 mit einem Markierelement 32 sein (Figur 3), mit dieser Tastspitze 31 lassen sich punktweise die Konturen des Femurs 20 bestimmen. Ein anderes Anlageelement wird durch ein L-Profil 33 mit einem Markierelement 34 gebildet. Das L-Profil weist an seiner Innenseite zwei senkrecht zueinander stehende, ebene Anlageflächen 35, 36 auf, die beispielsweise so am distalen bzw. dorsalen Ende des Femurs angelegt werden können, daß die am distalen Ende anliegende Anlagefläche 35 senkrecht auf der zuvor bestimmten mechanischen Achse des Femurs 3 steht (Figur 3). Auf diese Weise läßt sich die Erstreckung der Gelenkflächen 24 und 25 des Femurs 3 bestimmen.

Aus den auf diese Weise bestimmten Konturdaten kann man für jede Relativposition von Femur und Tibia den Abstand der Gelenkflächen 24 und 25 von der Sägeebene 21 berechnen, und zwar sowohl medial als auch lateral.

Ohne daß bisher am Femur irgendeine Veränderung vorgenommen worden ist, werden jetzt in einem nächsten Arbeitsschritt die Bewegungsabläufe simuliert, die sich im Kniegelenk ergeben würden, wenn eine Endogelenkprothese einer bestimmten Geometrie in bestimmten angenommenen Positionen implantiert wird. Man geht also zunächst von einem bestimmten Typ einer Kniegelenkendoprothese aus, die beispielsweise aus einem Tibiateil, einem Zwischenteil und einem Femurteil besteht, die Abmessungen dieser Teile sind bekannt, ebenso die Relativbewegungen der Teile dieser Endoprothese relativ zueinander, die sich aufgrund der geometrischen Ausgestaltung der Endogelenkprothese bestimmen lassen. Diese Daten werden in Form eines Datensatzes beispielsweise über die Tastatur 16 in die Datenverarbeitungsanlage eingegeben.

Unter der Annahme, daß das Tibiateil mit einer entsprechenden Anlagefläche an der Sägeebene 21 dicht anliegt, kann man auf diese Weise berechnen, wie sich bei einer Bewegung der Endogelenkprothese entsprechende Anlageflächen am Femurteil bewegen. Diese Anlageflächen des Femurteils entsprechen Sägeebenen am Femur, die zur Anpassung des Femurs an das Femurteil angebracht werden müssen. Je nach Lage dieser Sägeebenen wird der Femur unterschiedlich relativ zum Femurteil positioniert, und dies führt natürlich zu unterschiedlichen Relativpositionen von Femur und Tibia beim Bewegungsablauf der Knieendogelenkprothese.

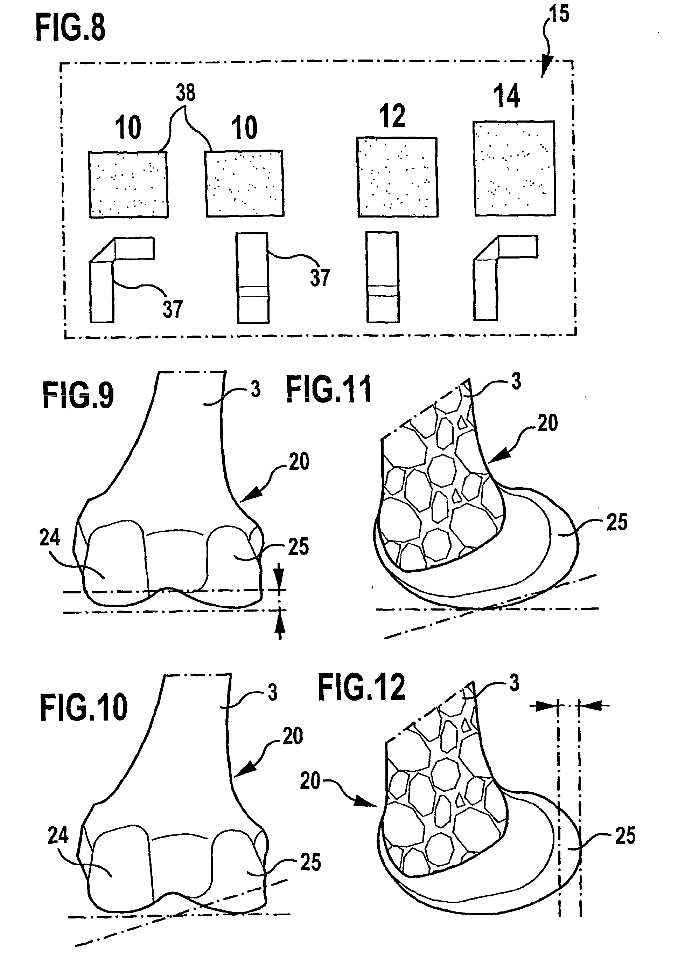

Die Lage des Femurteils relativ zum Femur kann beispielsweise dadurch variiert werden, daß das Femurteil in Richtung der mechanischen Achse des Femurs unterschiedlich positioniert wird (Figur 9), es ist möglich, daß das Femurteil gegenüber dem Femur geneigt wird, beispielsweise um eine anterior-posterior verlaufende Mittelachse (Figur 10) oder um eine medial-lateral verlaufende Mittelachse (Figur 11) das Femurteil kann auch in anterior-posteriorer Richtung verschoben werden (Figur 12). Dies führt zu einer entsprechenden Veränderung von Anlageflächen des Femurteils an dem entsprechend vorbereiteten Femur, dies ist in den Figuren 9 bis 12 durch strichpunktierte Linien dargestellt, die unterschiedliche Positionen dieser Anlageflächen zeigen.

Für jede Relativposition des Femurteils relativ zum Femur kann die Datenverarbeitungsanlage 14 die Relativposition von Femur und Tibia bei gestrecktem und bei abgewinkeltem Knie berechnen und damit z.B. auch die Breite des Spaltes 30 bei gestrecktem und bei abgewinkeltem Knie an der medialen und an der lateralen Gelenkfläche.

Der Operateur hat damit die Möglichkeit, unterschiedliche Relativpositionen des Femurteils gegenüber dem Femur anzunehmen und mit dieser angenommenen Position die Auswirkungen auf die Positionierung von Femur und Tibia relativ zueinander zu berechnen und damit auch beispielsweise die Breiten des Spaltes 30 für eine ganz bestimmte angenommene Position des Femurteils relativ zum Femur.

Um eine solche spezielle Position annehmen zu können, ist es beispielsweise sinnvoll, am Femur eine Sägeschablone zu befestigen und diese Sägeschablone relativ zum Femur dann durch geeignete Verstellmöglichkeiten unterschiedlich zu positionieren. Die durch die Sägeschablone definierte Ebene kann beispielsweise die distale Sägefläche des Femurs definieren, diese definierte Sägeebene kann durch Verstellung der Sägeschablone relativ zum Femur verschoben oder verschwenkt werden. Wenn die Sägeschablone ebenfalls mit einem Markierelement verbunden ist, kann somit diese angenommene Sägeebene räumlich bestimmt werden, d.h. das Navigationssystem ermittelt die Lage dieser angenommenen Sägeebene relativ zum Femur.

Der Operateur hat dadurch die Möglichkeit, diese Sägeebene in unterschiedlichste Relativpositionen zum Femur zu bringen und dann mit dieser angenommenen Stellung zu berechnen, wie sich Femur und Tibia aufgrund der Kinematik der Endoprothese relativ zueinander von der abgewinkelten bis in die gestreckte Stellung bewegen. Bei jeder angenommenen Sägeebene und damit jeder angenommenen Position des Femurteils relativ zum Femur ergeben sich somit beispielsweise Daten über die laterale und mediale Breite des Spaltes 30 bei gestrecktem und abgewinkeltem Knie. Diese Daten können auf der Anzeige 15 sichtbar gemacht werden, wie dies schematisch in Figur 8 gezeigt ist. Dort sind die Spaltbreiten in Form von Rechtecken nebeneinander dargestellt, und zwar auf einer Seite für die mediale Gelenkfläche und auf der anderen Seite für die laterale Gelenkfläche, in beiden Fällen für abgewinkeltes und für gestrecktes Knie. Die Strecklage wird durch Symbole 37 gekennzeichnet, die Spaltbreite durch senkrechte Balken 38, deren Höhe der Spaltbreite entspricht. Diese Höhe ist zusätzlich noch in Zahlen angegeben.

Auf der Anzeige 15 kann zusätzlich ein Bild des Femurs dargestellt werden, in weiches die Lage der angenommenen Sägeebene eingeblendet wird, so daß der Benutzer sofort erkennen kann, wie diese angenommene Sägeebene relativ zum Femur angeordnet ist. Dies ist insbesondere dann von Bedeutung, wenn diese Sägeebene geneigt wird, der Neigungswinkel gegenüber einer senkrecht auf der mechanischen Achse des Femurs stehenden Fläche kann dann sofort abgelesen werden. Damit kann der Operateur auch überprüfen, welche angenommenen Positionen er auswählt, um beispielsweise eine Varus-Valgus-Korrektur zu erreichen.

Wenn der Operateur also durch Verstellung der Sägeschablone eine bestimmte angenommene Position des Femurteils am Femur vorgibt, erscheinen auf der Anzeige unverzüglich die Breiten des Spaltes 30 für die laterale und die mediale Gelenkfläche sowohl für gestrecktes als auch für abgewinkeltes Knie, ohne daß irgendeine Bewegung des Knies dazu notwendig wäre. Die auf diese Weise erreichten Spaltbreiten kann der Operateur nunmehr mit den Spaltbreiten vergleichen, die er erreichen will, sei es, daß diese Spaltbreiten genau den Spaltbreiten entsprechen, die er vorher bei dem patienteneigenen Knie mit Hilfe des Distraktionsgerätes 26 bestimmt hat, sei es, daß er von diesen Werten in genau definierter Weise abweichen will, beispielsweise um unterschiedliche mediale und laterale Spaltbreiten einander anzugleichen.

Bei dieser Gelegenheit kann der Operateur auch Auswirkungen auf das Spannungsverhalten der Seitenbänder überprüfen, die sich dadurch ergeben, daß er bestimmte Fehlstellung des Knies korrigieren will, beispielsweise eine Varus-Valgus-Fehlstellung. Er kann somit einen Kompromiß zwischen Stellungskorrektur einerseits und Eingriff in das Spannungsverhalten der Seitenbänder anderseits schließen und die optimale Lage des Femurteils relativ zum Femur vorab bestimmen, ohne daß dazu der Femur in irgendeiner Weise bearbeitet werden müßte.

Zusätzlich hat der Operateur natürlich auch die Möglichkeit, dieses Verfahren für eine Endoprothese mit unterschiedlichen Abmessungen oder unterschiedlichem kinematischem Verhalten durchzuführen, auch dadurch ergeben sich unterschiedliche Bewegungen von Femur und Tibia relativ zueinander. Es besteht also durch Eingabe der entsprechenden Datensätze in die Datenverarbeitungsanlage 14 zusätzlich die Möglichkeit, auch noch unterschiedliche Prothesenteile und deren Auswirkungen auf den Bewegungsablauf zu simulieren, so daß einerseits über die Auswahl der Prothesenteile und andererseits über die angenommene Position der Prothesenteile relativ zu Femur und/oder Tibia der gesamte zu erwartende Bewegungsablauf simuliert und mit den gewünschten Ergebnissen verglichen werden kann.

Grundsätzlich wäre es auch möglich, eine entsprechende Berechnung für unterschiedliche angenommene Positionen des Tibiateils relativ zum Tibiakopf durchzuführen, es ist aber vorteilhaft, wenn in der beschriebenen Weise von einer vorgegebenen Sägeebene 21 für eine bestimmte Position des Tibiateils ausgegangen wird und die Variation im wesentlichen dadurch erfolgt, daß die Prothese selbst und/oder die angenommene Position des Femurteils relativ zum Femur variiert werden.

Sobald eine Lage der Sägeebene gefunden ist, die optimal ist, kann mit Hilfe der Sägeschablone ein entsprechender Schnitt am Femur durchgeführt werden, beispielsweise wird die distale Sägefläche ausgebildet, daran schließen sich dann weitere Sägeflächen an, beispielsweise eine dorsale Sägefläche, die senkrecht auf der distalen Sägefläche steht. Deren Lage wird in ganz ähnlicher Weise dadurch bestimmt, daß unterschiedliche Lagen dieser Sägefläche angenommen und dann die Auswirkung der unterschiedlichen Positionierung auf die Kinematik berechnet wird. Zum Beispiel führt die Verschiebung der dorsalen Schnittebene dazu, daß das Femurteil relativ zum Femur in anterior-posteriorer Richtung unterschiedlich positioniert wird.

Wenn die Schnitte ausgeführt sind, wird deren genaue Lage noch einmal dadurch verifiziert, daß navigierte Anlageelemente an die Schnittebenen angelegt werden, beispielsweise das Anlageelement 22, so daß sichergestellt ist, daß die erzeugte Sägeebene auch tatsächlich der ausgewählten, angenommenen Position der Sägeebene entspricht.

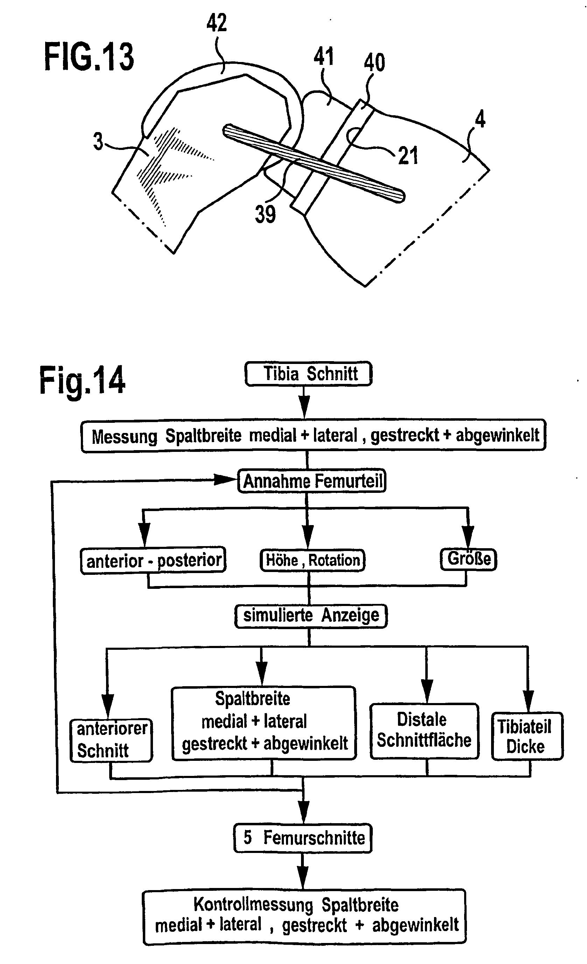

Die geschilderten Verfahrensschritte sind in der Darstellung der Figur 14 schematisch zusammengefaßt, insbesondere erkennt man daraus, daß der Operateur den Simulationsvorgang mit unterschiedlichen angenommenen Positionen des Femurteils relativ zum Femur mehrfach wiederholt, bis die optimale Lage gefunden ist.

In Figur 13 ist schematisch dargestellt, wie Tibiateil 40, Zwischenteil 41 und Femurteil 42 der Knieendogelenkprothese nach erfolgter Implantatation an Tibia und Femur angeordnet sind, man erkennt in dieser Darstellung auch schematisch, wie Tibia und Femur durch Seitenbänder 39 gegeneinander gespannt werden.

## Patentansprüche

1. System zur Bestimmung der Lage des Tibiateiles (40) und/oder des Femurteils (42) einer Kniegelenkendoprothese relativ zum proximalen Tibiakopf (19) bzw. zum distalen Femur (20) mit einem Navigationssystem (12) zur Überwachung der Lage des Femurs (3) und der Tibia (4) über an diesen festlegbare Markierelemente (7, 8), mit einem Distraktionsgerät (26), welches den distalen Femur (20) und den proximalen Tibiakopf (19) bei gestrecktem und bei abgewinkeltem Knie lateral und medial mit einer definierten Kraft in eine gespreizte Position verschiebt, mit einer Datenverarbeitungsanlage (14), die die Relativpositionen von Femur (3) und Tibia (4) bei dieser Distraktion und damit die Größe des Spaltes (30) zwischen Femur (3) und Tibia (4) bestimmt und die in Abhängigkeit von geometrischen Daten der Kniegelenkendoprothese und verschiedenen angenommenen Positionen des Tibiateils (40) an der Tibia (4) und/oder des Femurteils (42) am Femur (3) verschiedene virtuelle Relativpositionen von Femur (3) und Tibia (4) bei gestrecktem und gebeugtem Knie berechnet.

2. System nach Anspruch 1, **dadurch gekennzeichnet, daß** der Datenverarbeitungsanlage (14) eine Anzeige (15) zugeordnet ist, die der Relativposition von Femur (3) und Tibia (4) bei der Distraktion entsprechende Daten und den virtuellen Relativpositionen entsprechende Daten zum Zwecke von deren Vergleich anzeigt.

3. System nach Anspruch 2, **dadurch gekennzeichnet, daß** die Datenverarbeitungsanlage (14) auf der Anzeige (15) die Größe des medialen und lateralen Spaltes (30) in der gestreckten und in der abgewinkelten Stellung des Knies in den jeweils berechneten virtuellen Relativpositionen darstellt.

4. System nach Anspruch 2 oder 3, **dadurch gekennzeichnet, daß** die Datenverarbeitungsanlage (14) auf der Anzeige (15) die für die Bestimmung verschiedener virtueller Relativpositionen angenommene Position des Femurteils (42) relativ zu geometrischen Daten des Femurs (3) anzeigt.

5. System nach einem der Ansprüche 1 bis 4, **dadurch gekennzeichnet, daß** es mindestens ein navigiertes Anlageelement (22, 31, 33) umfaßt, welches zur Bestimmung der Größe des Spaltes (30) zwischen Femur (3) und Tibia (4) an die Kontur des proximalen Tibiakopfes (19) und die Kontur des distalen Femurs (20) anlegbar ist.

6. System nach Anspruch 5, **dadurch gekennzeichnet, daß** ein Anlageelement (31) eine Tastspitze aufweist, mit der verschiedene Punkte der Konturen abgetastet werden.

7. System nach Anspruch 5 oder 6, **dadurch gekennzeichnet, daß** ein Anlageelement (22, 33) eine ebene Anlagefläche aufweist, die man an die zu bestimmende Kontur anlegt.

8. System nach Anspruch 7, **dadurch gekennzeichnet, daß** ein Anlageelement (33) zwei senkrecht aufeinanderstehende Anlageflächen (35, 36) aufweist, die man gemeinsam an die zu bestimmende Kontur anlegt.

## Claims

1. System for determining the position of the tibial part (40) and/or the femoral part (42) of a knee-joint endoprosthesis in relation to the proximal tibial head (19) or to the distal femur (20) with a navigation system (12) for monitoring the position of the femur (3) and the tibia (4) by means of marking elements (7, 8) which can be fixed on the femur and tibia, with a distraction appliance (26), which displaces the distal femur (20) and the proximal tibial head (19) laterally and medially with a defined force into a spread position when the knee is straightened and bent, with a data processing system (14), which determines the relative position of the femur (3) and the tibia (4) during the distraction; and consequently the size of the gap (30) between the femur (3) and the tibia (4), and calculates various virtual relative positions of the femur (3) and the tibia (4) according to geometrical data of the knee-joint endoprosthesis and different assumed positions of the tibial part (40) on the tibia (4) and/or of the femoral part (42) on the femur (3) when the knee is straightened and bent.

2. System according to Claim 1, **characterized in that** a display (15) which displays data corresponding to the relative position of the femur (3) and the tibia (4) during the distraction and data corresponding to the virtual relative positions for the purpose of their comparison is associated with the data processing system (14).

3. System according to Claim 2, **characterized in that** the data processing system (14) shows on the display (15) the size of the medial and lateral gaps (30) in the straightened and bent positions of the knee in the respectively calculated virtual relative positions.

4. System according to Claim 2 or 3, **characterized in that** the data processing system (14) displays on the display (15) the position of the femoral part (42) assumed for the determination of various virtual relative positions in relation to geometrical data of the femur (3).

5. System according to one of Claims 1 to 4, **characterized in that** it comprises at least one navigated engaging element (22, 31, 33), which can be placed against the contour of the proximal tibial head (19) and the contour of the distal femur (20) to determine the size of the gap (30) between the femur (3) and the tibia (4).

6. System according to Claim 5, **characterized in that** an engaging element (31) has a contact tip, with which various parts of the contours are sensed.

7. System according to Claim 5 or 6, **characterized in that** an engaging element (22, 33) has a planar engagement surface, which is placed against the contour to be determined.

8. System according to Claim 7, **characterized in that** an engaging element (33) has two mutually perpendicular engagement surfaces (35, 36), which are jointly placed against the contour to be determined.

## Revendications

1. Système destiné à déterminer la position d'un élément tibial (40) et/ou d'un élément fémoral (42) d'une endoprothèse du genou par rapport à la tête de tibia proximale (19) ou par rapport au fémur distal (20), au moyen d'un système de navigation (12) destiné à contrôler la position du fémur (3) et du tibia (4) par l'intermédiaire d'éléments de repère (7, 8) propres à être fixés à ceux-ci, comportant un dispositif d'élongation (26) par lequel le fémur distal (20) et la tête de tibia proximale (19) sont déplacés en latéral et en médial avec une force définie dans une position écartée, lorsque le genou est en extension et en flexion, comportant un dispositif de traitement des données (14), qui détermine les positions relatives du fémur (3) et du tibia (4) au moment de cette élongation et, de ce fait, la dimension de la fente (30) entre le fémur (3) et le tibia (4) et qui, en fonction de données géométriques de l'endoprothèse du genou et de différentes positions supposées de l'élément tibial (40) sur le tibia (4) et/ou de l'élément fémoral (42) sur le fémur (3), calcule différentes positions relatives virtuelles du fémur (3) et du tibia (4) lorsque le genou est en extension ou en flexion.

2. Système selon la revendication 1, **caractérisé en ce qu'**au dispositif de traitement des données (14) est associé un dispositif d'affichage (15), qui affiche des données correspondant à la position relative du fémur (3) et du tibia (4) lors de l'élongation et des données correspondant aux positions relatives virtuelles afin de les comparer les unes aux autres.

3. Système selon la revendication 2, **caractérisé en ce que** le dispositif de traitement des données (14) représente sur le dispositif d'affichage (15) la dimension de la fente (30) médiale et latérale dans la position du genou en extension et en flexion, dans les positions relatives virtuelles calculées dans chaque cas.

4. Système selon la revendication 2 ou 3, **caractérisé en ce que** le dispositif de traitement des données (14) affiche sur le dispositif d'affichage (15) la position de l'élément fémoral (42), supposée pour la détermination des différentes positions relatives virtuelles, par rapport aux données géométriques du fémur (3).

5. Système selon l'une quelconque des revendications 1 à 4, **caractérisé en ce qu'**il comporte au moins un élément de contact (22, 31, 33) navigué qui, pour déterminer la dimension de la fente (30) entre le fémur (3) et le tibia (4), peut être amené en appui sur le contour de la tête de tibia proximale (19) et le contour du fémur distal (20).

6. Système selon la revendication 5, **caractérisé en ce qu'**un élément de contact (31) comporte une pointe de palpation, qui permet de palper différents points des contours.

7. Système selon la revendication 5 ou 6, **caractérisé en ce qu'**un élément de contact (22, 33) comporte une surface de contact plane, qui est amenée en appui sur le contour à déterminer.

8. Système selon la revendication 7, **caractérisé en ce qu'**un élément de contact (33) comporte deux surfaces de contact (35, 36) perpendiculaires l'une à l'autre, qui sont amenées en appui conjointement sur le contour à déterminer.
